Europäisches Patentamt

European Patent Office

Office européen des brevets

(11) Publication number: **0 212 853**
**A2**

# (12) EUROPEAN PATENT APPLICATION

(21) Application number: 86305602.4

(22) Date of filing: 22.07.86

(51) Int. Cl.⁴ **A61K 31/415** , A61K 9/08 , A61K 47/00

(30) Priority: 21.08.85 US 767997

(43) Date of publication of application:
04.03.87 Bulletin 87/10

(84) Designated Contracting States:
AT BE CH DE FR GB IT LI LU NL SE

(71) Applicant: WARNER-LAMBERT COMPANY
201 Tabor Road
Morris Plains New Jersey 07950(US)

(72) Inventor: Kao, Shui-Hsi
8 Kentwood Road
Succasunna New Jersey 07876(US)
Inventor: Kay, Allen I.
112 Righter Road
Succasunna New Jersey 07876(US)
Inventor: Sampson, Karla
15 Elmwood Terrace
West Caldwell New Jersey 07006(US)

(74) Representative: Jones, Michael Raymond et al
HASELTINE LAKE & CO. Hazlitt House 28
Southampton Buildings Chancery Lane
London WC2A 1AT(GB)

(54) Parenteral phenytoin compositions.

(57) Stable, aqueous solutions or phenytoin or phenytoin sodium are disclosed, which employ, as a stabilising agent, polyvinylpyrrolidone, with or without an alcoholic solvent system.

EP 0 212 853 A2

## PARENTERAL PHENYTOIN COMPOSITIONS

Phenytoin is 5,5-diphenyl-2,4-imidazolidinedione. It is a well-known pharmaceutical agent having anti-convulsant and anti-epileptic activity. Its preparation is described in U.S. Patent No. 2,409,754. Phenytoin and phenytoin sodium are well-known compounds. They are described in The Merck Index, 10th ed. (1983) on pages 1054 and 1-55.

Owing to phenytoin's relatively poor solubility in water, phenytoin is usually employed in the form of phenytoin sodium in the preparation of injectable solutions of the drug. In order to stabilize solutions of phenytoin sodium, it has been conventional to employ aqueous alcoholic solvent systems. Such solvent systems generally contain considerable quantities of propylene glycol and/or other alcohols.

Even in the presence of such alcoholic solvents, these solutions can be unstable following dilution in intravenous fluids, in that crystals form therein, precluding their safe use.

Thus, the need arose for stable aqueous preparation based on phenytoin or phenytoin sodium, with or without solvents, which can be used in combination with one or more conventional intravenous fluid(s).

According to one aspect of the present invention, there is provided an injectable composition containing a mixture of about from 1 to 10 wt.% of phenytoin or phenytoin sodium and about from 3 to 17 wt.% of polyvinylpyrrolidone, the weight percentages being based on the injectable solution.

The present invention is based on the discovery that aqueous solutions of phenytoin and phenytoin sodium, especially the latter, can be stabilized against undesirable crystal formation by the addition of suitable amounts of polyvinylpyrrolidone (hereinafter referred to as PVP).

In a preferred embodiment of the aforementioned aspect of the present invention, about 40 to 50 mg/ml aqueous phenytoin or phenytoin sodium is mixed with about from 3 to 17% PVP.

Weight percentages are based on the total composition weight, unless stated otherwise.

The use of alcoholic solvent systems along with PVP is optional. When such a solvent system is used, preferably about 40 wt.% of propylene glycol and about 10 wt.% of ethyl alcohol can be added to the PVP-containing mixture.

The pH of the final solution to be subsequently diluted with I.V. fluid, such as 0.9% sodium chloride injection of 5% dextrose injection, should be in the range of about 10 to about 13, preferably about pH 12.

In use of the injectable solution of the present invention, the phenytoin or phenytoin sodium anticonvulsant can be administered by the administration of a material containing phenytoin or phenytoin sodium and PVP in a weight ratio of about 1:0.5 to about 1:3.5.

The solutions, preparations and materials in accordance with the present invention generally exhibit several advantages which conventional phenytoin and phenytoin sodium-containing substances do not.

Phenytoin, because of its low solubility, cannot normally be effectively used in injectable solutions. It is extremely difficult to ensure uniform dosages when it is used alone in aqueous solutions.

Phenytoin sodium, on the other hand, is more soluble in water, but it rapidly dissociates into phenytoin, which then comes out of solution, resulting in precipitation especially during intravenous infusion. The preparations according to the present invention suffer from neither the low solubility associated with phenytoin nor the crystal formation usually associated with the conventional solutions of phenytoin sodium.

The high solubility of the phenytoin sodium/PVP combinations in water makes parenteral formulations containing them highly useful. For example, they can be diluted with suitable fluid(s) to make preparations suitable for intravenous infusion.

As indicated above, the present invention is concerned with novel solutions which result from the combination of phenytoin or phenytoin sodium with PVP in an aqueous environment. Whilst not wishing to be bound by this theory, it is expected that the PVP stabilizer achieves its effectiveness by nucleation inhibition.

By "aqueous environment" is meant water-containing systems which optionally include conventional diluents for phenytoin or phenytoin sodium. Such conventional diluents include alcoholic solvents, such as $C_{1-5}$ alcohols and $C_{2-6}$ diols. Ethanol and propylene glycol are preferred alcohols. Mixtures are contemplated. While alcoholic solvents are preferred, other diluents may be incorporated in the compositions so long as they do not detract from the solubility or stability of the phenytoin or phenytoin sodium.

Generally, the phenytoin sodium and PVP components will be brought together in aqueous solution. Preferred methods for preparing the combinations include:

(1) dissolving dry PVP in phenytoin or phenytoin sodium in aqueous solution;

(2) mixing an aqueous PVP solution with an aqueous phenytoin or phenytoin sodium solution; and

(3) dry mixing of PVP and phenytoin or phenytoin sodium, followed by solution in aqueous medium.

Other conventional solution techniques can be employed.

The PVP phenytoin or phenytoin sodium solutions are suitable for dilution with other fluids to render them useful for intravenous infusion.

The quantity of phenytoin or phenytoin sodium in these solutions will range from about 1 to 10 wt.%, and preferably about 4 to about 5wt.% based on the weight of the total composition (i.e. PVP, water and phenytoin or phenytoin sodium).

The quantity of PVP in these solutions will range from about 3 to about 17 wt.%, preferably from about 6 to about 10 wt.%, based on the total composition weight.

In general, the weight ratio of phenytoin sodium to PVP in these solutions and in the preparations subsequently produced therefrom will be about 1:0.5 to about 1:3.5, with about 1:2 preferred.

When a diol solvent, such as propylene glycol, is present, it will usually be used in an amount between about 20 to about 80 wt.%, and preferably about 40 wt.%.

When an monohydric alcohol solvent is used, e.g. ethanol, it will generally be present at about 5 to about 15 wt.%, and preferably about 10 wt.%, based on total composition weight.

Thus the weight ratio of diol to monohydric alcohol solvent components, when both are used, will generally be about 1:0.25 to about 1:0.75.

The compositions of the present invention -i.e., the combinations of phenytoin or phenytoin sodium, PVP, and optional alcoholic solvents -can be subjected to conventional purification, e.g. sterilization procedures. The may then be suitably packaged for storage and/or use, e.g. in ampoules, vials or syringes.

In order to adjust the pH of the compositions to the proper value, sodium hydroxide or other suitable alkaline material(s) is generally added thereto. If the pH of the solution exceeds the useful values, it can be lowered via the addition of hydrochloric acid or other suitable acidic substance(s).

The intravenous fluids with which the compositions of the invention are usually mixed include conventional fluids whose properties do not detract from the solubility of the phenytoin or phenytoin sodium or the polyvinylpyrrolidone components. In general, they are aqueous solutions containing conventional quantities of electrolytes and nutrient.

While these diluents are generally referred to as aqueous, they may contain as their principal constituents non-aqueous media, as long as those media are pharmaceutically acceptable and do not interfere with the solubility of the phenytoin or phenytoin sodium ingredient.

Useful intravenous fluids include aqueous solutions of saline and dextrose. Preferred saline solutions contain up to 0.9%, preferably 0.9 wt.%, sodium chloride in aqueous, preferably water, solution.

Useful dextrose solutions are those containing from about 1 to about 10 wt.%, preferably 5 wt.%, dextrose in aqueous, preferably water, solution.

The combining of the phenytoin or phenytoin sodium/PVP compositions with conventional fluids to make preparations suitable for administration via injection is effected by conventional methods. Simple mixing of the compositions with suitable quantities of the fluids is a preferred technique.

The present invention will now be illustrated by the following examples.

Example 1

750 Millilitres of water for injection, USP were heated to 40° -45°C in a water bath. 100 Grams of polyvinylpyrrolidone, USP were added thereto with vigorous mixing, until dissolved. The solution was cooled to room temperature in an ice water bath.

10 Millilitres of 1.0N aqueous NaOH solution were added to the cooled solution and mixed well. 50.-Grams of phenytoin sodium USP (adjusted to 100% purity based on assay) were added and mixed well. If necessary, the pH of the resulting mixture can be adjusted at this point with NaOH to a pH of about 12.1-12.3, preferably 12.2. A sufficient amount of water for injections was added to yield 980 ml. Mixing was continued. The pH may again be adjusted, if necessary, to pH 12.20 with NaOH solution.

Enough water for injection was added, with mixing, to bring the volume to 1000 ml.

The solution was sterilized by filtration through a previously sterilized Millipore assembly fitted with a Pall N66R membrane using nitrogen for positive pressure. 5.3-5.5 Millilitre aliquots of the solution were aseptically filled under nitrogen atmosphere into previously sterilized and depyrogenated ampoules. The filled ampoules were post-purged with high purity nitrogen, and then the ampoules were sealed.

The resultant solution was a light yellow clear solution having a tentative pH of 12.1-12.3; it is stable for at least three months in ampoules.

It is highly preferred that, as a precaution, the solutions be kept out of contact with $CO_2$; in addition, maintenance of the solution under nitrogen during preparation, filling and storage is highly desirable.

## Example 2

50 Grams of phenytoin sodium USP (adjusted to 100% purity based on assay) were mixed with about 275 ml of water for injection, USP. To this mixture was added 30.4 ml of 0.84% aqueous sodium hydroxide solution (made by combining 1.68g NaOH with 200 ml water for injection, USP). A slurry was produced.

To the slurry were added 400.0 ml propylene glycol, USP and 110.4 ml ethanol USP (5.0% excess of alcohol was included). Mixing was continued until a clear solution was formed. Polyvinylpyrrolidone, USP, was then added with vigorous mixing, until dissolved.

Any pH adjustment, if necessary, was made by adding NaOH to give a pH of about 12.

A sufficient amount of water for injection was added to bring the volume to 1,000 ml. The ingredients were mixed well.

The solution was sterilized by filtration through a previously sterilized Millipore assembly fitted with a Pall N66R membrane using nitrogen for positive pressure. Previously sterilized and depyrogenated Kimble 5cc Type I ampoules were aseptically filled under a nitrogen atmosphere, each with 5.1-5.3 ml of the solution. The filled ampoules were post-purged with high purity nitrogen, and then the ampoules were sealed.

The final composition was a light yellow clear solution having a tentative pH of about 12.1 to 12.3, preferably 12.2.

The final composition was stable for at least three months in ampoules and at least one month in vials.

The solutions produced in Example 1 are exemplary of solutions which can be produced using various concentrations of polyvinylpyrrolidone - (PVP) in a wholly aqueous system containing sodium hydroxide at a pH of about 12.0. As they are totally aqueous systems, they can be used for direct IV use at any rate which the physician desires, based on patients' needs.

In addition, the solutions produced in Examples 1 and 2 exhibit sufficient physical stability following dilution in either 5% Dextrose Injection, USP or 0.9% or 0.9% Sodium Chloride Injection, USP, to allow for the safe use of this product for IV infusion.

## Claims =

1. An injectable composition containing a mixture of about from 1 to 10 wt.% of phenytoin or phenytoin sodium and about from 3 to 17 wt.% of polyvinylpyrrolidone, the weight percentages being based on the injectable solution.

2. An injectable solution according to Claim 1, wherein the phenytoin or phenytoin sodium is present as from 4 to 5 wt.%.

3. An injectable solution according to Claim 1 or 2, wherein the polyvinylpyrrolidone is present as from 6 to 10 wt.%.

4. An injectable solution according to Claim 1, 2 or 3, wherein the ratio of phenytoin sodium or phenytoin to PVP is about from 1:0.5 to 1:3.5.

5. An aqueous preparation comprising an injectable composition according to any preceding claim, and at least one intravenous fluid.

6. An aqueous preparation according to Claim 5, wherein the intravenous fluid is saline solution.

7. An aqueous preparation according to Claim 5, wherein the intravenous fluid is dextrose solution.

8. An injectable composition according to any one of Claims 1 to 4, or an aqueous preparation according to Claim 5, 6 or 7, which also includes at least one alcoholic solvent selected from propylene glycol and ethanol.

9. A process for producing an aqueous preparation for the administration of phenytoin or phenytoin sodium, which comprises dissolving phenytoin or phenytoin sodium and polyvinylpyrrolidone in an aqueous solution.

10. An injectable composition according to any one of Claims 1 to 4 and 8, or an aqueous preparation according to any one of Claims 5 to 7 and 8, wherein phenytoin sodium is the anti-convulsant agent.

## Claims =

## Claims for contracting state : AT:

1. A process for producing an injectable composition containing a mixture of about from 1 to 10 wt.% of phenytoin or phenytoin sodium and about from 3 to 17 wt.% of polyvinylpyrrolidone, the weight percentages being based on the injectable solution, which process comprises dissolving phenytoin or phenytoin sodium and polyvinylpyrrolidone in an aqueous solution.

2. A process according to Claim 1, wherein the phenytoin or phenytoin sodium is present as from 4 to 5 wt.%.

3. A process according to Claim 1 or 2, wherein the polyvinylpyrrolidone is present as from 6 to 10 wt.%.

4. A process according to Claim 1, 2 or 3, wherein the ratio of phenytoin sodium or phenytoin to PVP is about from 1:0.5 to 1:3.5.

5. A process for producing an aqueous preparation, which comprises admixing an injectable composition as produced according to any preceding claim, and at least one intravenous fluid.

6. A process according to Claim 5, wherein the intravenous fluid is saline solution.

7. A process according to Claim 5, wherein the intravenous fluid is dextrose solution.

8. A process injectable composition according to any preceding claim, which also includes at least one alcoholic solvent selected from propylene glycol and ethanol.

9. A process according to any preceding claim, wherein phenytoin sodium is the anti-convulsant agent.